# EUROPEAN PATENT SPECIFICATION

(11) **EP 2 176 270 B1**
(45) Date of publication and mention of the grant of the patent: **29.05.2019**
(21) Application number: 08786558.0
(22) Date of filing: 29.07.2008
(51) Int. Cl.: C07D 499/42, C07D 501/18

(54) **COMPOSITIONS COMPRISING BETA LACTAM COMPOUNDS**
BETA-LACTAM- VERBINDUNGEN ENTHALTENDE ZUSAMMENSETZUNGEN
COMPOSITIONS COMPRENANT DES COMPOSÉS BÊTA-LACTAMES

(30) Priority: 30.07.2007 EP 07113423
(43) Date of publication of application: 21.04.2010
(73) Proprietor: Centrient Pharmaceuticals Netherlands B.V., 2613 AX Delft (NL)
(72) Inventor: PLUGGE, Willem, NL-2645 MG Delfgauw (NL); KUIPERS, Karel Hendrik, NL-2612 CC Delft (NL); ZALM, VAN DER, Johannes Albert, NL-2681 JM Monster (NL)
(74) Representative: de Pauw, Elmar Sebastian David
(86) International application number: PCT/EP2008/059920
(87) International publication number: WO 2009/016171

(56) References cited:
- EP-A- 0 001 133
- US-A- 3 249 622
- US-A- 3 992 428

## Description

The present invention relates to compositions comprising at least 95 wt% of a β-lactam compound as well as to a process for their production and a process for the production of semi-synthetic antibiotics.

Synthetic routes to prepare semi-synthetic penicillins (SSP's) and semi-synthetics cephalosporins (SSC's) usually start from fermentation products such as penicillin G (PenG), penicillin V (PenV) and/or Cephalosporin C (CefC). These compounds may be converted to the corresponding β-lactam intermediates such as 6-aminopenicillanic acid (6-APA), 7-amino-desacetoxy-cephalosporanic acid (7-ADCA), 7-aminocephalosporanic acid (7-ACA), 7-amino-3-chloro-3-cephem-4-carboxylate (7-ACCA), 7-amino-3-[(Z/E)-1-propen-1 -yl]-3-cephem-4-carboxylate (7-PACA) and others as is disclosed in K. Matsumoto, Bioprocess. Techn., 16, (1993), 67-88, J.G. Shewale & H. Sivaraman, Process Biochemistry, August 1989, 146-154, T.A. Savidge, Biotechnology of Industrial Antibiotics (Ed. E.J. Vandamme) Marcel Dekker, New York, 1984, or J.G. Shewale et al., Process Biochemistry International, June 1990, 97-103.

For instance 6-APA may be obtained from PenG or PenV via chemical or enzymatic cleavage of the phenylacetic acid side or hydroxyphenylacetic acid side chain respectively. Pen G and PenV are both secondary metabolites produced and excreted by the filamentous fungus *Penicillium chrysogenum* in large scale industrial fermentation processes (see R.P. Elander in Appl. Microbiol. Biotechnol (2003) 61:385-392 for a review). In some of these processes, PenG is produced in a crystalline form as an intermediate, in other processes, this step is avoided and the side chain is cleaved off directly in an aqueous PenG-solution obtained after extraction and back extraction of the Pen-G from the fermentation broth - see WO98/48039.

The first generation 7-ADCA product was derived from PenG whereby both the expansion of the 5-membered penem ring to the 6-membered cephem ring and the subsequent cleavage of the phenylacetic acid side chain of the phenylacetyl-7-ADCA were carried out using chemical reactions. The next generation 7-ADCA product was still obtained from PenG but after the chemical ring expansion, the phenylacetic acid side chain of the phenylacetyl-7-ADCA was cleaved off enzymatically using a suitable (penicillin) acylase. Other processes have been developed wherein also the ring expansion of PenG to phenylacetyl-7-ADCA is carried out in vitro using a suitable expandase enzyme, but these processes are of little industrial importance.

The most recent and most elegant production process for 7-ADCA comprises the culturing of a *Penicillium chrysogenum,* transformed with and expressing a gene encoding a suitable expandase. This engineered *Penicillium chrysogenum* strain, when grown in the presence of adipic acid as the side chain precursor in the fermentation vessel, produces and excretes adipyl-7-ADCA - see WO93/05158. In this production process, the adipyl-7-ADCA is recovered from the fermentation broth, subjected to a suitable acylase to cleave of the adipic acid side chain after which the 7-ADCA thus obtained is further purified, crystallized and dried. Other side chains precursors have been disclosed in WO95/04148 (2-(carboxyethylthio)acetic acid and 3-(carboxymethylthio)-propionic acid), WO95/04149 (2-(carboxyethylthio)propionic acid), WO96/38580 (phenyl acetic acid) and WO98/048034 and WO98/048035 (various dicarboxylic acids). The expandase takes care of the expansion of the 5-membered ring of the various N-acylated penicillanic acids, thereby yielding the corresponding N-acylated desacetoxycephalosporanic acids.

Production of other β-lactam intermediates such as 7-ACA, 7-ACCA and 7-PACA has been amply documented in the patent and scientific literature and are known to the skilled person.

The β-lactam intermediates are subsequently converted to the desired SSP or SSC by coupling to a suitable side chain, as has been described in *inter alia* EP 0 339 751, JP-A-53005185 and CH-A-640 240. By making different combinations of side chains and β -lactam intermediates, a variety of semi-synthetic penicillin and cephalosporin antibiotics may be obtained such as amoxicillin, ampicillin, cephalexin, cefadroxil, cephradine, cefaclor and cefprozil. In the case of the chemical coupling of the side chain to a β-lactam intermediate, the carboxyl group of the β-lactam intermediate is first silylated in in order to promote the dissolution of the insoluble β-lactam intermediate in the organic solvent system.

The dissolution rate of a β-lactam intermediate composition is dependent on many factors such as particle size, purity of the β-lactam intermediate composition, absence or presence of impurities or water, temperature, type of organic solvent etceteras. We have now surprisingly found that compositions comprising at least 90% of a β-lactam intermediate and further containing between 0.02 and 5 wt% ammonium chloride and/or a corresponding amount of the HCI salt of an organic amine (based on molecular weight), have superior dissolution rates compared to compositions not containing ammonium chloride and/or a corresponding amount of the HCl salt of an organic amine.

EP-A-0001133 describes a process for the preparation of silylated 6-aminopenicillanic acids such as 6-APA (see e.g. example Ib) and 7-amino-cephalosporinic acids such as 7-ADCA (see e.g. examples XVIa and XVIIIa) comprising the silylation of the 6-APA and 7-ADCA with trimethylchlorosilane (TMCS) or hexamethyldisilazan (HMDS) in a solvent such as dichloromethane and (in the case of TMCS silylation) in the presence of a tertiary amine such as triethylamine. EP-A-0001133 does not disclose a composition containing ammonium chloride and/or HCl salt of an organic amine component.

US-A-3992428 discloses a process for the preparation of bis- (trimethylsilyl) -urea by reacting urea with HMDS (silylating agent) in the presence of 0.1 to 5 wt% (see claim 2) of e.g. an ammonium salt such as ammonium chloride (see example 2). US-A-3992428 is not related to compositions comprising 6-APA, 7-ADCA, 7-ACA, 7-ACCA and 7-PACA or the production thereof. US-A-3249622 discloses a process for preparation of silylated 6-APA with HDMS in the presence of 0.55 g of ammonium sulfate (see column 2, lines 17-58).

In a first aspect, the invention provides a composition comprising at least 90 wt% of a β-lactam compound characterized in that it contains between 0.02 and 5 wt% ammonium chloride and/or a corresponding amount of the HCI salt of an organic amine (based on molecular weight), wherein the β-lactam compound is a β-lactam intermediate, which is selected from the group consisting of 6-APA, 7-ADCA, 7-ACA, 7-ACCA and 7-PACA. More preferably, the β-lactam intermediate is 6-APA or 7-ADCA, most preferably the β-lactam intermediate is 7-ADCA.

The composition of the invention comprises at least 90%, preferably at least 95%, more preferably at least 96%, more preferably at least 97%, more preferably at least 98% and most preferably at least 99% of the β-lactam intermediate. The ammonium chloride content of the composition of the invention is between 0.02 and 5 wt%, more preferably between 0.02 and 4 wt%, more preferably between 0.03 and 3 wt%, more preferably between 0.04 and 2 wt% and most preferably between 0.05 and 1wt%. Likewise, embodiments of the invention comprising the HCl salt of an organic amine, have a content of the HCl salt of an organic amine which is preferably in amounts corresponding to those of ammonium chloride (based on molecular weight). Embodiments containing both ammonium chloride and the HCl salt of an organic amine, contain amounts that correspond to those stated for ammonium chloride alone (based on molecular weights).

The advantage of the composition of the invention is that it combines a very high purity with respect to the β-lactam intermediate and improved dissolution behaviour in silylation reactions, compared to a composition not containing between 0.02 and 5 wt% ammonium chloride and/or a corresponding amount of the HCl salt of an organic amine (based on molecular weight).

The composition of the invention may further comprise water. Preferably, the water content is equal to or less than 0.5 wt%, more preferably equal to or less than 0.4 wt%, more preferably equal to or less than 0.3 wt% and most preferably equal to or less than 0.2 wt%.

For the preferred embodiment wherein the β-lactam intermediate is a cephalosporin intermediate selected from the group consisting of 7-ACA, 7-ADCA, 7-ACCA and 7-PACA, the composition may preferably have a very low 6-APA content. For oral administration of the semi-synthetic cephalosporins, the 6-APA content in the composition of the invention comprising a cephalosporin intermediate selected from the group consisting of 7-ACA, 7-ADCA, 7-ACCA and 7-PACA is preferably less than 50 ppm, more preferably less than 40 ppm, more preferably less than 30 ppm, more preferably less than 20 ppm and most preferably less than 10 ppm. For parental administration, the 6-APA content in the composition of the invention comprising a cephalosporin intermediate selected from the group consisting of 7-ACA, 7-ADCA, 7-ACCA and 7-PACA, is preferably less than 10 ppm.

For the preferred embodiment wherein the β-lactam intermediate is a cephalosporin intermediate selected from the group consisting of 7-ACA, 7-ADCA, 7-ACCA and 7-PACA, the composition of the invention preferably does not contain phenylacetic acid. This means that, by applying analytical methods known in the art for the detection of phenylacetic acid to determine the content of phenylacetic acid in the composition of the invention, the response of said analytical methods remains below the detection limit for said compounds. This means that the composition of the invention comprising a cephalosporin intermediate selected from the group consisting of 7-ACA, 7-ADCA, 7-ACCA and 7-PACA as the β-lactam intermediate preferably comprises equal to or less than 4 ppm phenylacetic acid.

In a second aspect the invention provides a process for the production of the composition of the invention comprising contacting a composition comprising the β-lactam compound selected from the group consisting of 6-APA, 7-ADCA, 7-ACA, 7-ACCA and 7-PACA and which does not contain ammonium chloride and/or the HCl salt of an organic amine with ammonium chloride and/or the HCl salt of an organic amine. The β-lactam compound is a β-lactam intermediate, which is selected from the group consisting of 6-APA, 7-ADCA, 7-ACA, 7-ACCA and 7-PACA. More preferably, the β-lactam intermediate is 6-APA or 7-ADCA, most preferably the β-lactam intermediate is 7-ADCA. Preferably, the composition comprising at least 90%, preferably at least 95% of the β-lactam compound and which does not contain ammonium chloride and/or the HCl salt of an organic amine is in crystalline form and is washed one or more times with an aqueous solution of ammonium chloride and/or the HCl salt of an organic amine and subsequently optionally dried. In a preferred embodiment, the crystals are washed one or several times with one or more bed volumes of the aqueous solution of ammonium chloride and/or the HCl salt of an organic amine in a suitable concentration. These concentrations preferably are between 0.25 and 5 wt%, more preferably between 0.5 and 2.5 wt%. Drying of the washed crystals may be carried out according to methods known in the art until a water content is reached as described hereinbefore for the composition of the invention.

In a third aspect, the invention provides a process for the production of semi-synthetics antibiotics comprising silylating the composition of the invention. For the production of semi-synthetic penicillins, the composition of the invention comprises 6-APA, while for the production of semi-synthetic cephalosporins, the composition of the invention may comprise any of the cephalosporin intermediates selected from the group consisting of 7-ADCA, 7-ACA, 7-ACCA and 7-PACA.

In a fourth aspect, the invention provides the use of the composition of the invention for the chemical synthesis of semi-synthetic β-lactam antibiotics. The composition of the invention comprising 6-APA as the β-lactam intermediate may be used for the production of semi-synthetic penicillins, while the composition of the invention comprising a cephalosporin intermediate, selected from the group consisting of 7-ADCA, 7-ACA, 7-ACCA and 7-PACA may be used for the production of semi-synthetic cephalosporins. Preferred semi-synthetic penicillins are ampicillin and amoxicillin. Preferred semi-synthetic cephalosporins are cephalexin, cefadroxil, cephradine, cefaclor and cefprozil.

### EXAMPLES

### Example 1

### Preparation of a composition comprising 7-ADCA and ammonium chloride

Adipyl-7-ADCA was produced in a fermentation process using an engineered *Penicillium chrysogenum* strain as described in WO93/05158. The adipyl-7-ADCA was recovered, deacylated to 7-ADCA and the 7-ADCA crystallized as described in WO98/48035 and WO98/48036. Five litres of an aqueous suspension of 7-ADCA crystals (between 40-50 g/L 7-ADCA) was filtered over a G3 filter with a diameter of 13 cm using a vacuum pump. The 7-ADCA crystals were rinsed twice with one bed volume of a 1.5 wt% aqueous solution of ammonium chloride (NH₄Cl). After the second rinse, the crystals were dried on the G3 filter for 20 minutes. Approximately 100 gram of the crystals was dried for 2 hours in an oven at 102°C. The bed volume was calculated from the height of the crystal cake on the filter and the diameter of the filter.

## Claims

1. A composition comprising at least 90 wt% of a β-lactam compound selected from the group consisting of 6-APA, 7-ADCA, 7-ACA, 7-ACCA and 7-PACA **characterized in that** it contains between 0.02 and 5 wt% ammonium chloride and/or a corresponding amount of the HCl salt of an organic amine.

2. A composition according to claim 1 wherein the β-lactam compound is 7-ADCA.

3. A process for the production of any of the compositions of claims 1-2 comprising contacting the composition comprising at least 95 wt% of a β-lactam compound and which does not contain ammonium chloride and/or the HCl salt of an organic amine with ammonium chloride and/or the HCl salt of an organic amine.

4. A process according to claim 3 wherein a composition comprising at least 95 wt% of a β-lactam compound in crystalline form is washed one or more times with an aqueous solution of ammonium chloride and/or the HCl salt of an organic amine and optionally dried.

5. A process for the production of semi-synthetic antibiotics comprising silylation of any of the compositions of claims 1-2.

6. Use of the composition as described in any of claims 1-2 for the production of semi-synthetic β-lactam antibiotics.

## Patentansprüche

1. Zusammensetzung, umfassend mindestens 90 Gew.-% einer β-Lactam-Verbindung aus der Gruppe bestehend aus 6-APA, 7-ADCA, 7-ACA, 7-ACCA und 7-PACA, **dadurch gekennzeichnet, dass** sie zwischen 0,02 und 5 Gew.-% Ammoniumchlorid und/oder einer entsprechenden Menge des HCl-Salzes eines organischen Amins enthält.

2. Zusammensetzung nach Anspruch 1, wobei es sich bei der β-Lactam-Verbindung um 7-ADCA handelt.

3. Verfahren zur Herstellung einer der Zusammensetzungen der Ansprüche 1-2, bei dem man die Zusammensetzung, die mindestens 95 Gew.-% einer β-Lactam-Verbindung umfasst und die kein Ammoniumchlorid und/oder HCl-Salz eines organischen Amins enthält, mit Ammoniumchlorid und/oder dem HCl-Salz eines organischen Amins in Kontakt bringt.

4. Verfahren nach Anspruch 3, bei dem man eine Zusammensetzung, die mindestens 95 Gew.-% einer β-Lactam-Verbindung in kristalliner Form umfasst, ein- oder mehrmals mit einer wässrigen Lösung von Ammoniumchlorid und/oder dem HCl-Salz eines organischen Amins wäscht und gegebenenfalls trocknet.

5. Verfahren zur Herstellung von halbsynthetischen Antibiotika, bei dem man eine der Zusammensetzungen der Ansprüche 1-2 silyliert.

6. Verwendung der Zusammensetzung gemäß einem der Ansprüche 1-2 zur Herstellung von halbsynthetischen β-Lactam-Antibiotika.

## Revendications

1. Composition comprenant au moins 90 % en poids d'un composé β-lactame choisi dans le groupe constitué de 6-APA, 7-ADCA, 7-ACA, 7-ACCA et 7-PACA, **caractérisée en ce qu'**elle contient entre 0,02 et 5 % en poids de chlorure d'ammonium et/ou une quantité correspondante du sel de HCl d'une amine organique.

2. Composition selon la revendication 1, dans lequel le composé β-lactame est 7-ADCA.

3. Procédé de production selon l'une quelconque des compositions selon les revendications 1 à 2, comprenant la mise en contact de la composition comprenant au moins 95 % en poids d'un composé de β-lactame et qui ne contient pas de chlorure d'ammonium et/ou le sel de HCl d'une amine organique avec du chlorure d'ammonium et/ou le sel de HCl d'une amine organique.

4. Procédé selon la revendication 3 dans lequel une composition comprenant au moins 95 % en poids d'un composé de β-lactame sous forme cristalline est lavée une ou plusieurs fois avec une solution aqueuse de chlorure d'ammonium et/ou du sel de HCl d'une amine organique et facultativement séchée.

5. Procédé de production d'antibiotiques semi-synthétique comprenant la silylation selon l'une quelconque des compositions selon les revendications 1 à 2.

6. Utilisation de la composition telle que décrite dans l'une quelconque des revendications 1 à 2 pour la production d'antibiotiques β-lactames semi-synthétiques.
